Europäisches Patentamt

European Patent Office

Office européen des brevets

(19)

(11) **EP 0 954 280 B1**

(12) **FASCICULE DE BREVET EUROPEEN**

(45) Date de publication et mention
de la délivrance du brevet:
**27.11.2002 Bulletin 2002/48**

(21) Numéro de dépôt: **97952097.0**

(22) Date de dépôt: **18.12.1997**

(51) Int Cl.⁷: **A61K 7/48**, A61K 35/78

(86) Numéro de dépôt international:
**PCT/FR97/02342**

(87) Numéro de publication internationale:
**WO 98/027956 (02.07.1998 Gazette 1998/26)**

(54) **UTILISATION D'UN EXTRAIT DE TERMINALIA CATAPPA**

VERWENDUNG EINES TERMINALIA CATAPPA EXTRAKTES

USE OF AN EXTRACT OF TERMINALIA CATAPPA

(84) Etats contractants désignés:
**AT BE CH DE DK ES FI FR GB GR IE IT LI LU MC
NL PT SE**

(30) Priorité: **20.12.1996 FR 9615793**

(43) Date de publication de la demande:
**10.11.1999 Bulletin 1999/45**

(73) Titulaire: **PARFUMS CHRISTIAN DIOR**
**75008 Paris (FR)**

(72) Inventeurs:
• **RENIMEL, Isabelle**
**F-45470 Trainou (FR)**
• **OLIVIER, Marc**
**F-30113 Les Angles (FR)**
• **ANDRE, Patrice**
**F-45170 Neuville aux Bois (FR)**
• **Cabalion, Pierre**
**98848 Noumea Cedex, Nouvelle-Caledone (FR)**

(74) Mandataire: **Giraud, Françoise**
**Cabinet Beau de Loménie**
**158, rue de l'Université**
**75340 Paris Cedex 07 (FR)**

(56) Documents cités:
**EP-A- 0 283 713        WO-A-98/10740**

• **STN, Serveur de Bases de Données,
XP002037510**
• **STN, Serveur de Bases de Données,
XP002037511**
• **STN, Serveur de Bases de Données,
XP002037512**

**Description**

L'invention concerne des utilisations d'un extrait de la plante Terminalia catappa, dans les domaines cosmétique et pharmaceutique, notamment dermatologique.

Elle concerne plus précisément des utilisations comme agent cosmétique d'un extrait de cette plante.

La plante Terminalia catappa est une plante de la famille des Combretaceae que l'on trouve en particulier en Nouvelle Calédonie. WO-A-98 10740 concerne l'utilisation d'un extrait de la plante Terminalia catappa, seul ou mélangé avec d'autres composés actifs, dans une composition cosmétique, dermatologique et/ou pharmaceutique à usage topique externe pour la peau et les phanères.

Les essais systématiques réalisés par les inventeurs ont permis de mettre en évidence un certain nombre d'actions enzymatiques surprenantes des extraits de cette plante, en particulier des actions d'inhibition de plusieurs enzymes, en particulier, la phospholipase $A_2$ et la 5'-lipoxygénase, de la tyrosinase et de la 3',5' AMPc-phosphodiestérase, ce qui a permis d'envisager son utilisation dans des produits cosmétiques et pharmaceutiques, notamment dermatologiques.

La phospholipase $A_2$ ($PLA_2$) est une enzyme produite par les cellules au niveau membranaire. Elle prédomine dans les cellules liées aux phénomènes d'inflammation, telles que les mastocytes. Par son action, elle libère l'acide arachidonique lié aux phospholipides membranaires. Cet acide se métabolise ensuite en différents médiateurs lipidiques de l'inflammation et de l'allergie, tels que les leucotriènes et les prostaglandines.

La 5'-lipoxygénase, désignée ci-après par "lipoxygénase", est, comme la $PLA_2$, une enzyme membranaire. Elle intervient dans la "cascade de l'inflammation" en aval de la libération de l'acide arachidonique par la $PLA_2$, dans la conversion de cet acide en leucotriènes, médiateurs de l'inflammation.

La 3',5'-AMPc-phosphodiestérase, désignée ci-après "phosphodiestérase" ou "PDE", est l'enzyme qui transforme l'AMPc, second messager qui intervient dans le contrôle du métabolisme cellulaire, en AMP inactif. L'inhibition de la PDE par un agent inhibiteur permet en conséquence de maintenir un taux intracellulaire élevé d'AMPc, ce qui a pour effet notamment d'activer les protéines kinases A, et, par ce processus, permet de favoriser la dégradation des lipides.

De plus, il est également connu que l'AMPc intervient pour contrer certains processus inflammatoires (M. Hitchcock, J. Immunol. (1977) 188 557). Egalement, il a été décrit que la phosphodiestérase augmente avec l'âge (S. K. Puri et L. Volicer, Mechanisms of Aging and Dev. (1981) 15 239). De ce fait, son inhibition contribuera à lutter contre les effets du vieillissement, en particulier au niveau cutané.

La tyrosinase est l'enzyme clé de la synthèse de la mélanine et donc du métabolisme de la pigmentation cutanée. En cosmétique, son inhibition, par des agents appropriés, trouve des applications dans le traitement local des hyperpigmentations cutanées, telles que les tâches pigmentaires de sénescence.

Ainsi, il a été mis en évidence par les inventeurs, que, grâce à leur action inhibitrice sur les enzymes précitées, les extraits de la plante Terminalia catappa présentaient un grand intérêt en cosmétique et en thérapeutique.

En effet, par la découverte de l'activité des extraits de la plante Terminalia catappa, inhibant l'action d'enzymes, l'invention fournit différentes solutions dans le domaine de la cosmétique et de la thérapeutique, notamment de la dermatologie. S'agissant de l'inhibition de la phospholipase $A_2$ d'une part et de la lipoxygénase d'autre part, les compositions selon l'invention agissent ainsi doublement dans le processus de formation des médiateurs de l'allergie cutanée et de l'inflammation, en limitant ou en bloquant ce processus.

L'inhibition de la phosphodiestérase (PDE) conduit à une action amincissante, anti-inflammatoire et anti-vieillissement des compositions de l'invention. L'inhibition de la tyrosinase leur confère un effet dépigmentant de la peau.

D'autres avantages de l'invention apparaissent au vu de la description et des exemples qui suivent.

Ainsi, selon l'une de ses caractéristiques essentielles, l'invention concerne des compositions cosmétiques contenant un extrait de la plante Terminalia catappa en présence d'un véhicule cosmétiquement acceptable.

Ce sont essentiellement les feuilles qui s'avèrent intéressantes pour la préparation des extraits de l'invention.

L'extrait est avantageusement obtenu par macération de la plante ou partie de plante dans un solvant ou un mélange de solvants, suivie d'une filtration. Le solvant de la solution obtenue peut être au besoin évaporé pour obtenir l'extrait sec.

De préférence, l'évaporation sera réalisée sous pression réduite.

A titre de solvants avantageusement utilisés, on citera :

- l'eau
- des solvants chlorés, notamment le dichlorométhane
- les éthers, tels que l'éther éthylique ou diisopropylique
- l'acétone
- les esters en $C_2$ à $C_8$ tels que l'acétate d'éthyle et l'acétate de butyle

- des alcools en $C_1$ à $C_6$, tels que le méthanol, l'éthanol et l'isopropanol
- des polyols en $C_2$ à $C_6$, tels que le propylèneglycol ou le glycérol.

On pourra également obtenir l'extrait de la plante par la technique dite de l'extraction au dioxyde de carbone supercritique.

Selon un mode de réalisation avantageux, cette composition comprend de 0,001 à 10 % en poids et en particulier de 0,02 à 1 % en poids d'extrait sec de la plante par rapport au poids total de la composition finale.

Par ailleurs, les essais réalisés par les inventeurs ont clairement montré que, non seulement le rendement d'extraction était lié à la nature du solvant utilisé mais également l'activité enzymatique de l'extrait. Les exemples joints en annexe montrent clairement l'effet du choix du solvant sur l'activité enzymatique de l'extrait.

Les compositions selon l'invention peuvent être formulées selon toute forme acceptable pour leur emploi en cosmétologie. Il peut, en particulier, s'agir d'une composition sous forme appropriée pour une application topique, en particulier sous forme d'une crème ou d'un gel, en particulier d'une crème ou d'un gel pour le visage, les mains, le buste ou le corps.

Selon un autre aspect, l'invention concerne l'utilisation de l'extrait de plante comme agent cosmétique, ledit agent étant incorporé dans une composition cosmétique telle que définie précédemment.

Cet agent cosmétique sera notamment utilisé dans toutes les applications où l'on recherche, en particulier, à inhiber l'action et/ou de la phospholipase $A_2$ et/ou de la lipoxygénase, et/ou de la phosphodiestérase et/ou de la tyrosinase.

Elles pourront également être utilisées pour obtenir un amincissement de différentes parties du corps, en particulier des hanches. Elles pourront également être destinées à atténuer ou à faire disparaître les tâches pigmentaires cutanées.

Ainsi, les compositions cosmétiques de l'invention seront notamment utilisées pour toutes les applications cosmétiques où l'on cherche à inhiber l'activité des enzymes précitées.

Elles s'avèrent également intéressantes pour leur action amincissante ou leur action dépigmentante.

Comme on l'a vu précédemment, l'efficacité des compositions cosmétiques précédemment décrites a pu être corrélée avec un certain nombre d'activités enzymatiques. La mise en évidence de ces activités enzymatiques a permis d'envisager également l'utilisation des extraits définis précédemment pour la préparation de compositions pharmaceutiques, notamment dermatologiques dans lesquelles de telles activités sont souhaitées. Les essais réalisés par les inventeurs de la présente invention ont confirmé l'efficacité de ces compositions pharmaceutiques.

Ainsi, l'inhibition de la phospholipase $A_2$ et l'inhibition de la 5'-lipoxygénase ont pu être corrélées avec une efficacité dans la prévention et le traitement des phénomènes d'inflammation.

L'inhibition de la tyrosinase a pu être corrélée avec une action dans le traitement local d'une hyperpigmentation de la peau.

L'inhibition de la PDE se traduit par le maintien d'un taux élevé d'AMPc intra-cellulaire. Ceci conduit à divers effets intéressant la peau, en particulier des effets amincissants, anti-vieillissement et anti-inflammatoire.

Ainsi, selon une autre caractéristique essentielle, l'invention concerne également l'utilisation d'un extrait de la plante Terminalia catappa pour la préparation d'une composition pharmaceutique, notamment dermatologique, destinée au traitement des effets du vieillissement intrinsèque ou actinique de la peau, à la prévention ou au traitement des manifestations cutanées des allergies et des inflammations, à la réduction des surcharges graisseuses souscutanées, au traitement des tâches cutanées pour les atténuer ou les faire disparaître, ledit extrait étant incorporé dans un véhicule pharmaceutiquement acceptable.

Pour ces différentes applications, la composition pharmaceutique présente avantageusement une activité inhibitrice de la phospholipase $A_2$ et/ou de la lipoxygénase et/ou de la phosphodiestérase et/ou de la tyrosinase.

Selon une variante, l'invention concerne l'utilisation d'un extrait de Terminalia catappa pour la préparation d'une composition pharmaceutique, notamment dermatologique destinée au traitement et à la prévention des manifestations allergiques, en particulier de l'allergie cutanée.

Ce type d'application est directement lié à l'inhibition des médiateurs lipidiques de l'inflammation.

Dans toutes les applications du domaine pharmaceutique, notamment dermatologique, les compositions utilisées sont de préférence des compositions à application topique destinées à être appliquée sur la peau. Par ailleurs, les extraits de la plante utilisés pour leur préparation sont obtenus de la même façon que les extraits utilisés dans le domaine cosmétique, à partir des mêmes parties de plante et sont introduits dans un véhicule pharmaceutiquement, notamment dermatologiquement, acceptable à des concentrations comprises entre 0,001 % et 10 % en poids, et en particulier entre 0,02 % et 1 % en poids, d'extrait sec de ladite plante ou partie de plante.

Comme dans le cas des applications cosmétiques, on choisira le solvant d'extraction en fonction du type d'activité enzymatique que l'on souhaitera privilégier dans l'action de la composition pharmaceutique.

Les exemples qui suivent sont donnés à titre purement illustratif de l'invention.

Sauf indication contraire, les proportions données dans les exemples de compositions sont exprimées en pourcentage en poids

Exemple 1

Préparation d'un extrait selon l'invention

1 g de feuilles de la plante Terminalia catappa préalablement séchées et broyées est introduit dans 200 ml de solvant. La suspension est laissée, à température ambiante sous agitation modérée, pendant 4 heures. On filtre ensuite le mélange, puis on évapore le solvant du filtrat ainsi obtenu sous pression réduite. On récupère alors l'extrait sec. Pour préparer les compositions selon l'invention, il est possible d'utiliser soit la solution d'extrait de plante, éventuellement concentrée, dans le solvant d'extraction, soit l'extrait sec.
Des extraits ont été réalisés en utilisant 2 solvants différents : l'eau et le méthanol.

Exemple 2

Mise en évidence de l'activité inhibitrice des extraits sur différentes enzymes

2.1 Extrait utilisés

Les tests d'inhibition enzymatiques étant menés en milieu aqueux, il est nécessaire d'utiliser des solvants d'extraction miscibles avec l'eau.
Ainsi, en procédant comme décrit dans l'exemple 1, on a réalisé trois extraits différents avec respectivement de l'eau, du méthanol et du DMSO. La concentration de ces extraits est ensuite ajustée à 0,5 % en extrait sec de plante, soit par ajout soit par évaporation du solvant d'extraction.
Tous les tests décrits ci-après ont été réalisés en trois exemplaires. Les valeurs rapportées sont des moyennes arithmétiques.

2.2. Inhibition de l'élastase

a) Principe du test :

Les techniques de mise en évidence de l'inhibition de l'élastase ont été décrites par différents auteurs (BAUMSTARK, J.S. et al., Biochim. Biophys. Acta (1963), 77, 676 ; BIETH, B. et al., Biochem. Med., (1974), 11, 350 ; C. FRANCK, I. BYRJALSEN, Biol. Chem. Hoppe Seyler, (1988) 369 (8) 677-82).
Le principe actif est le suivant : un substrat est mis en présence d'élastase en milieu aqueux, puis, après incubation, on effectue une mesure des produits de la réaction.
En l'occurrence, le substrat est le N-succinyl-(Ala)$_3$-para-nitroanilide, disponible auprès de la Société Sigma (Réf. : S 4760) en solution à 0,5 mg/ml dans un tampon Tris-HCl 0,2 M à pH 8,8. L'élastase ajoutée au milieu réactionnel libère la para-nitroaniline et le résidu peptidique. L'évolution de la réaction est observée au spectrophotomètre Uvikon 941® (Kontron S.A.) à la longueur d'onde λ de 379 nm.
La composition du milieu réactionnel est la suivante :

- solution de substrat (0,5 mg/ml de tampon) :     200 μl
- tampon Tris-HCl :     600 μl
- solvant d'extraction :     100 μl

Le solvant d'extraction contient ou non l'effecteur, c'est-à-dire l'extrait de la plante Terminalia catappa à la concentration de 0,5 % en poids, selon qu'il s'agit de l'essai avec effecteur, ou de l'essai sans effecteur (activité basale de l'enzyme).
A ces 900 μl de milieu réactionnel, on ajoute extemporanément 100 μl de la solution d'enzyme à raison de 35 U/ml dans le tampon Tris-HCl.
On mesure alors la cinétique de la libération de para-nitroaniline par l'absorption d'une lumière mono-chromatique de longueur d'onde 379 nm au moyen du spectrophotomètre, ce qui permet de calculer le pourcentage d'inhibition $I_E$ suivant la formule -ci-dessous :

$$I_E = \frac{\Delta AbB / mn - \Delta AbE / mn}{\Delta AbB / mn} \times 100$$

dans laquelle $\Delta$AbB / mn est la différence d'absorbance par minute du milieu réactionnel pour l'activité basale, et $\Delta$AbE / mn, la différence d'absorbance du milieu réactionnel pour l'essai avec effecteur. Les valeurs d'absorbance prises en compte sont celles correspondantes à la période linéaire de l'évolution de l'absorbance en fonction du temps.

Les résultats figurent au tableau I ci-après.

## 2.3. Inhibition de la phospholipase A$_2$

Selon le principe du test (UTHE, J.F. and MAGEE, W.L., Can. J. Biochem. (1971), 49, 776 ; DENNIS, E.A., J. Lipids Res., (1973), 14, 152), l'enzyme est mise en présence d'un phospholipide. Celui-ci est alors transformé en lysolécithine avec libération d'un acide gras, insoluble dans le milieu réactionnel. Cette réaction peut donc être suivie par turbidimétrie au moyen d'un spectrophotomètre, en utilisant par exemple la longueur d'onde de 360 nm.

La composition du milieu réactionnel est indiquée ci-après. Toutes les solutions sont réalisées dans de l'eau distillée.

- solution à 14 mM de L-phosphatidylcholine dimyristoyl (substrat) : 600 µl
- solution de chlorure de sodium 0,67 M : 150 µl
- solution de chlorure de calcium 66 mM : 200 µl
- eau distillée : 850 µl
- solvant, sans ou avec effecteur à 0,5 % : 100 µl

A ce milieu, on ajoute extemporanément 100 µl de solution d'enzyme dans l'eau distillée à la concentration de 96 U/ml.

Comme dans le cas de l'inhibition de l'élastase, la cinétique réactionnelle est suivie au moyen d'un spectrophotomètre Uvikon 941®, à la longueur d'onde de 360 nm.

On détermine le pourcentage d'inhibition I$_p$ de la phospholipase A$_2$ par calcul suivant une formule analogue à celle de l'inhibition de l'élastase

$$I_p = \frac{\Delta AbB / mn - \Delta AbE / mn}{\Delta AbB / mn} \times 100$$

dans laquelle $\Delta$AbB et $\Delta$AbE ont la même signification que précédemment.

Les résultats figurent au tableau I ci-après.

## 2.4. Inhibition de la 5'-lipoxygénase

Comme cela a été exposé plus haut, la lipoxygénase intervient dans la formation de médiateurs de l'inflammation, en particulier les leucotriènes, à partir de l'acide arachidonique.

Dans l'essai de mise en évidence de l'inhibition de cette enzyme (C.W. Taylor, H.R. Morris, Brit. Med. Bull. (1989) 39 219-222 ; J. Magee, Methods of Enzymatic Analysis, H.U. Bergmayer Ed., (1965) p. 411-4, Academic Press, N.Y.), on utilise de l'acide linoléique comme substrat qui se transforme en présence de l'enzyme en acide 5-hydroperoxy-6,8,11,14-éicosatétraénoïque (5 HPETE).

Composition du milieu réactionnel :

- acide linoléique (substrat), solution 0,5 mM : 2 000 µl
- tampon borate 0,2 M à pH 9 : 950 µl
- solvant, sans ou avec effecteur à 0,5 % : 100 µl

dans le tampon borate

Pour préparer la solution d'acide linoléique ci-dessus, on réalise préalablement une salification partielle de l'acide linoléique dans du méthanol en présence de soude, puis on l'introduit dans le tampon borate.

On ajoute ensuite extemporanément 100 µl de solution de l'enzyme 5'-lipoxygénase dans du tampon borate à la concentration de 10 U/ml.

Comme dans les essais précédents, on mesure l'absorbance des milieux en fonction du temps, et on calcule, pour la partie linéaire de l'évolution de l'absorbance, le pourcentage d'inhibition I$_L$ de la 5'-lipoxygénase.

Les résultats figurent au tableau I ci-après.

2.5 Inhibition de la phosphodiestérase (PDE)

Le principe de ce test est basé sur l'hydrolyse de la 3',5'-adénosine monophosphate cyclique (AMP$_c$) en adénosine monophosphate (AMP). La formation d'AMP est mesurée par analyse HPLC.

La composition du milieu réactionnel est indiquée ci-dessous. Les solutions des réactifs sont réalisées dans du tampon Tris-HCl 0,05 M à pH 7,5.

- solution d'AMP$_c$ (substrat) à 0,25% dans le tampon          80 µl
- solvant, sans ou avec effecteur à 0,5%          80 µl
- tampon Tris-HCl          480 µl

A ce milieu, on ajoute extemporanément 160 µl de PDE à 0,5 U/ml dans le tampon Tris-HCl.

Au temps t = 5 minutes, on mesure la quantité d'AMP formée en calculant la surface d'intégration du pic de l'AMP sur le chromatogramme fourni par l'appareil HPLC (KONTION S.A.).

On peut alors estimer le taux d'inhibition de la PDE I$_A$ par l'effecteur suivant la formule :

$$I_A = \frac{SAMP_h - SAMP_e}{SAMP_b} \times 100$$

dans laquelle SAMP$_b$ représente la surface d'intégration du pic de l'AMP pour l'activité basale de l'enzyme (sans effecteur) et SAMP$_e$ représente la surface d'intégration du pic de l'AMP pour l'activité de l'enzyme en présence de l'effecteur.

Les résultats obtenus figurent en Tableau I ci-après.

2.6 Inhibition de la tyrosinase

Le principe de ce test est basé sur la formation de dopachrome à partir de la L-tyrosine, par l'action de la tyrosinase.

Il est rappelé que la L-tyrosine, en présence de tyrosinase et d'oxygène, s'oxyde en L-DOPA qui s'oxyde à son tour, encore par l'action de la tyrosinase, en dopaquinone. Celle-ci se cyclise alors en cyclodopa, qui s'oxyde en dopachrome, précurseur de mélanines et qui absorbe la lumière à la longueur d'onde de 480nm.

$$\text{L-tyrosine} \xrightarrow{\text{tyrosinase}} \text{L-DOPA} \xrightarrow{\text{tyrosinase}} \text{dopachrome} \ (\lambda = 480 \ nm)$$
$$\downarrow$$
$$\text{mélanines}$$

La formation de dopachrome peut donc être suivie par spectrophotométrie.

Pour cette méthode, on pourra se reporter en particulier aux publications de S.H. Pomerantz. Arch. Biochem. Biophys. (1974) 160 73-82, ou de J. Barber, J. Invest. Dermatol. (1984) 83 145-149.

La composition du milieu réactionnel est indiquée ci-dessous. Les solutions des réactifs sont réalisées dans du tampon phosphate 0,02 M à pH 6,9.

- solution de L-tyrosine (1er substrat) à 1 mM dans le tampon          333 µl
- solution de L-DOPA (2ème substrat) à 1 mM dans le tampon          333 µl
- solvant, sans ou avec effecteur à 0,5%          333 µl

A ce milieu réactionnel, on ajoute extemporanément 33 µl de solution de tyrosinase à la concentration de 2400 U/ml dans le tampon phosphate.

On mesure alors la cinétique de la formation de dopachrome par l'absorption d'une lumière monochromatique de longueur d'onde 480nm au moyen d'un spectrophotomètre Uvikon 941 (KONTION S.A.), ce qui permet de calculer, pour la partie linéaire de l'évolution de l'absorbance en fonction du temps, le pourcentage d'inhibition I$_T$ de la tyrosinase suivant la formule ci-dessous :

$$I_T = \frac{\Delta\ AbB/mn\ -\ \Delta\ AbE/mn}{\Delta\ AbB/mn}\ X\ 100$$

dans laquelle $\Delta AbB/mn$ est la différence d'absorbance par minute du milieu réactionnel pour l'activité basale de l'enzyme (sans effecteur), et $\Delta AbE/mn$, la différence d'absorbance du milieu réactionnel pour l'essai avec effecteur.

Les résultats figurent au Tableau I ci-après.

## TABLEAU I

### Taux d'inhibition enzymatique par les extraits de l'invention

| | Extraits | | |
|---|---|---|---|
| | $E_{eau}$ | $E_{méthanol}$ | $E_{DMSO}$ |
| $I_E$ (élastase) | 8 | 1 | 15 |
| $I_P$ (PLA2) | 85 | 36 | 68 |
| $I_L$ (lipoxygénase) | - | 19 | 73 |
| $I_T$ (tyrosinase) | 40 | - | - |
| $I_A$ (PDE) | 89 | 90 | 66 |

$E_{eau}$ : extrait aqueux, à 0,5 %

$E_{méthanol}$ : extrait méthanolique, à 0,5 %

$E_{DMSO}$ : extrait du DMSO, à 0,5 %

Les résultats figurant au Tableau I ci-dessus démontrent l'intérêt des extraits selon l'invention, dans les domaines de la cosmétique et de la pharmacie, notamment en dermatologie, dès qu'il s'agit de bloquer, de diminuer l'importance ou de réguler l'action de certaines enzymes.

Plus précisément, il ressort clairement que chacune des cinq enzymes testées, à l'exception cependant de l'élastase, est inhibée de manière importante par au moins l'un des extraits testés.

L'action des extraits de Terminalia catappa est particulièrement importante sur l'inhibition de la phosphodiestérase et de la phospholipase A2. Cependant, l'extrait aqueux est également actif sur l'inhibition de la tyrosinase, et l'extrait de DMSO est très actif sur celle de la lipoxygénase.

De ce fait, les extraits de Terminalia catappa peuvent être avantageusement utilisés pour les différentes applications citées plus haut découlant de l'inhibition des enzymes concernées.

Exemple 3

Crème pour peau sensible

- Extrait sec de feuilles de Terminalia catappa,
  fraction méthanolique selon l'exemple 1        0,2 g
- Méthylglucose sesquistéarate        3,0 g
- Cire d'abeille        3,0 g
- Alcool béhénique        3,0 g
- Octanoate d'octyle        5,0 g
- Huile minérale fluide        7,5 g
- Octanoate de cétostéaryle        5,0 g
- Glycérine        3,0 g
- Gomme xanthane        0,50 g
- Parfums        0,30 g
- Conservateur, colorants        qs
- Eau        qsp        100 g

Cette crème est utilisée pour le raffermissement de la peau du visage et du cou, tout en limitant les risques d'apparition de rougeurs ou de picotements.

Exemple 4

| Crème amincissante | | |
|---|---|---|
| Extrait sec selon l'exemple 1, faction méthanolique | | 0,5 g |
| Tampon phosphate | | 29,5 g |
| Excipient émulsionné eau-dans-huile (avec conservateur et parfum) | qsp | 100 g |

La crème ainsi obtenue est appliquée une à deux fois par jour sur les parties du corps à traiter, telles que les hanches, par cure d'une à trois semaines.

Exemple 5

| Gel dépigmentant | | |
|---|---|---|
| Extrait sec selon l'exemple 1, fraction aqueuse | | 1 g |
| Tampon phosphate | | 49 g |
| Excipient gel de Carbopol 940® à 4% neutralisé, avec conservateurs | qsp | 100 g |

Ce gel est utilisé en application locale sur les zones hyperpigmentées de la peau. Il est en particulier destiné à traiter les taches pigmentaires de senescence sur les mains, pour les atténuer ou les faire disparaître.

Exemple 6

| Gel dépigmentant | |
|---|---|
| Solution aqueuse d'extrait selon l'exemple 1, à 5% dans l'eau distillée | 4 g |
| Extrait d'écorce de mûrier | 0,35 g |

(suite)

| Gel dépigmentant | | |
|---|---|---|
| Acide ascorbique | | 0,6 g |
| Carbopol 940® | | 2 g |
| Eau + conservateurs | qsp | 100 g |

Ce gel est conseillé, en début de cure, pour traiter localement les taches pigmentaires cutanées telles que les taches de senescence.

**Revendications**

1. Utilisation d'un extrait de la plante Terminalia catappa pour la préparation d'une composition cosmétique, en tant qu'agent actif destiné à inhiber la phospholipase $A_2$ et/ou la lipoxygénase et/ou la phosphodiestérase et/ou la tyrosinase.

2. Utilisation selon la revendication 1, **caractérisée en ce que** ladite composition est destinée à inhiber la tyrosinase.

3. Utilisation selon la revendication 2, **caractérisée en ce que** ladite composition est destinée à atténuer ou à faire disparaître les taches pigmentaires cutanées.

4. Utilisation selon la revendication 1, **caractérisée en ce que** ladite composition est destinée à l'amincissement des différentes parties du corps, en particulier des hanches.

5. Utilisation d'un extrait de la plante Terminalia catappa pour la préparation d'une composition cosmétique, en tant qu'agent actif destiné à l'amincissement de différentes parties du corps, en particulier des hanches.

6. Utilisation d'un extrait de la plante Terminalia catappa pour la préparation d'une composition cosmétique en tant qu'agent actif destiné à atténuer ou à faire disparaître les taches pigmentaires cutanées.

7. Utilisation d'un extrait de la plante Terminalia catappa pour la préparation d'une composition pharmaceutique, notamment dermatologique, destinée à la prévention ou au traitement des manifestations cutanées des allergies et à la réduction des surchages graisseuses sous-cutanées, au traitement des taches cutanées pour les atténuer ou les faire disparaître, ledit extrait étant incorporé dans un véhicule pharmaceutiquement acceptable.

8. Utilisation selon la revendication 7, **caractérisée en ce que** la composition présente une activité inhibitrice de la phosphodipase $A_2$ et/ou de la lipoxygénase et/ou de la phosphodiestérase et/ou de la tyrosinase.

9. Utilisation selon l'une des revendications 1 à 8, **caractérisée par le fait que** ledit extrait est un extrait de feuilles de Terminalia catappa.

10. Utilisation selon l'une des revendications 1 à 9, **caractérisée en ce que** ledit extrait est obtenu par macération de ladite plante dans un solvant ou un mélange de solvants, suivie d'une filtration et, au besoin, d'une évaporation du solvant.

11. Utilisation selon la revendication 10, caractérisée en ce ledit solvant est choisi dans le groupe constitué par l'eau, les solvants chlorés, notamment le dichlorométhane, les éthers tels que l'éther éthylique ou diisopropylique, l'acétone, les esters en $C_2$ à $C_8$, tels que l'acétate d'éthyle et l'acétate de butyle, les alcools en $C_1$ à $C_6$, tels que le méthanol, l'éthanol et l'isopropanol, les polyols en $C_2$ à $C_6$, tels que le propylèneglycol et le glycérol, et leurs mélanges.

12. Utilisation selon l'une des revendications 1 à 9, **caractérisée en ce que** ledit extrait est obtenu par extraction au dioxyde de carbone supercritique.

13. Utilisation selon l'une des revendications 1 à 12, **caractérisée en ce que** ladite composition comprend de 0,001 % à 10 % en poids, et en particulier de 0,02 % à 1 % en poids, d'extrait sec de ladite plante.

**14.** Utilisation selon l'une des revendications 1 à 13, **caractérisée en ce que** ladite composition se présente sous toute forme appropriée pour une application topique, en particulier sous forme d'une crème ou d'un gel, en particulier d'une crème ou d'un gel pour le visage, les mains, le buste ou le corps.

**15.** Utilisation selon l'une des revendications 1 à 14, **caractérisée en ce que** ladite composition est formulée pour une application topique sur la peau.


**Patentansprüche**

**1.** Verwendung eines Extrakts der Pflanze Terminalia catappa für die Herstellung einer kosmetischen Zusammensetzung als aktives Mittel, bestimmt zur Inhibition der Phospholipase $A_2$ und/oder der Lipoxygenase und/oder der Phosphodiesterase und/oder der Tyrosinase.

**2.** Verwendung gemäss Anspruch 1, **dadurch gekennzeichnet, dass** die Zusammensetzung zur Inhibition der Tyrosinase bestimmt ist.

**3.** Verwendung gemäss Anspruch 2, **dadurch gekennzeichnet, dass** die Zusammensetzung dazu bestimmt ist, kutane Pigmentflecken abzuschwächen oder verschwinden zu lassen.

**4.** Verwendung gemäss Anspruch 1, **dadurch gekennzeichnet, dass** die Zusammensetzung dazu bestimmt ist, unterschiedliche Körperteile, insbesondere die Hüften, dünner werden zu lassen.

**5.** Verwendung eines Extrakts der Pflanze Terminalia catappa für die Herstellung einer kosmetischen Zusammensetzung als aktives Mittel, dazu bestimmt, unterschiedliche Körperteile, insbesondere die Hüften, dünner werden zu lassen.

**6.** Verwendung eines Extrakts der Pflanze Terminalia catappa für die Herstellung einer kosmetischen Zusammensetzung als aktives Mittel, bestimmt zum Abschwächen von kutanen Pigmentflecken oder um sie verschwinden zu lassen.

**7.** Verwendung eines Extrakts der Pflanze Terminalia catappa für die Herstellung einer pharmazeutischen Zusammensetzung, insbesondere einer dermatologischen, bestimmt zur Verhinderung oder Behandlung von kutanen Manifestationen von Allergien und für die Reduktion von subkutanen fettigen Überladungen, für die Behandlung von kutanen Flecken, um diese abzuschwächen oder sie verschwinden zu lassen, wobei der Extrakt in einen pharmazeutisch akzeptablen Träger inkorporiert ist.

**8.** Verwendung gemäss Anspruch 7, **dadurch gekennzeichnet, dass** die Zusammensetzung eine inhibitorische Wirkung der Phospholipase $A_2$ und/oder der Lipoxygenase und/oder der Phosphodiesterase und/oder der Tyrosinase aufweist.

**9.** Verwendung gemäss einem der Ansprüche 1 bis 8, **dadurch gekennzeichnet, dass** der Extrakt ein Extrakt der Blätter von Terminalia catappa ist.

**10.** Verwendung gemäss einem der Ansprüche 1 bis 9, **dadurch gekennzeichnet, dass** der Extrakt durch Maceration der Pflanze in einem Lösungsmittel oder in einer Mischung von Lösungsmitteln, gefolgt von einer Filtration und, falls nötig, einer Verdampfung des Lösungsmittels, erhalten wird.

**11.** Verwendung gemäss Anspruch 10, **dadurch gekennzeichnet, dass** das Lösungsmittel ausgewählt ist aus der Gruppe, gebildet durch Wasser, chlorierte Lösungsmittel, insbesondere Dichlormethan, Ether, wie z.B. Ethylether oder Diisopropylether, Aceton, $C_{2-8}$-Diester, wie z.B. Ethylacetat und Butylacetat, $C_{1-6}$-Alkohole, wie z.B. Methanol, Ethanol und Isopropanol, $C_{2-6}$-Polyole, wie z.B. Propylenglykol und Glycerin, und ihren Mischungen.

**12.** Verwendung gemäss einem der Ansprüche 1 bis 9, **dadurch gekennzeichnet, dass** der Extrakt durch Extraktion mit superkritischem Kohlendioxid erhalten wird.

**13.** Verwendung gemäss einem der Ansprüche 1 bis 12, **dadurch gekennzeichnet, dass** die Zusammensetzung 0,001 bis 10 Gew.% und insbesondere 0,02 bis 1 Gew.% des Trockenextrakts der Pflanze umfasst.

**14.** Verwendung gemäss einem der Ansprüche 1 bis 13, **dadurch gekennzeichnet, dass** sich die zusammensetzung in für eine topische Anwendung geeigneter Form präsentiert, insbesondere in Form einer Creme oder eines Gels, insbesondere einer Creme oder eines Gels für das Gesicht, die Hände, die Brust oder den Körper.

**15.** Verwendung gemäss einem der Ansprüche 1 bis 14, **dadurch gekennzeichnet, dass** die Zusammensetzung für eine topische Anwendung auf die Haut formuliert ist.

**Claims**

**1.** Use of an extract of the plant Terminalia catappa for the preparation of a cosmetic composition, as an active agent intended to inhibit phospholipase $A_2$ and/or lipoxygenase and/or phosphodiesterase and/or tyrosinase.

**2.** Use according to claim 1, **characterized in that** said composition is intended to inhibit tyrosinase.

**3.** Use according to claim 2, **characterized in that** said composition is intended for reducing or eliminating freckles.

**4.** Use according to claim 1, **characterized in that** said composition is intended for the slimming of different parts of the body, particularly the hips.

**5.** Use of an extract of the plant Terminalia catappa, for the preparation of a cosmetic composition, as an active agent intended for the slimming of different parts of the body, particularly of the hips.

**6.** Use of an extract of the plant Terminalia catappa, for the preparation of a cosmetic composition, as an active agent intended for reducing or eliminating freckles.

**7.** Use of an extract of the plant Terminalia catappa for the preparation of a pharmaceutical composition, especially dermatological composition, intended for the prevention or treatment of the skin manifestations of allergies and for reducing under-cutaneous fat overloads, for the treatment of freckles, in order to reduce or eliminate them, said extract being incorporated in a pharmaceutically acceptable vehicle.

**8.** Use according to claim 7, **characterized in that** said composition has an inhibitory activity on phospholipase $A_2$ and/or lipoxygenase and/or phosphodiesterase and/or tyrosinase.

**9.** Use according to one of claims 1 to 8, **characterized in that** said extract is an extract of the leaves of Terminalia catappa

**10.** Use according to one of claims 1 to 9, **characterized in that** said extract is obtained by maceration of said plant in a solvent or solvent mixture, followed by filtration and, if necessary, evaporation of the solvent.

**11.** Use according to claim 10, **characterized in that** said solvent is selected from the group comprising water, chlorinated solvents, especially dichloromethane, ethers such as ethyl or diisopropyl ether, acetone, $C_2$ to $C_8$ esters such as ethyl acetate and butyl acetate, $C_1$ to $C_6$ alcohols such as methanol, ethanol and isopropanol, $C_2$ to $C_6$ polyols such as propylene glycol and glycerol, and mixtures thereof.

**12.** Use according to one of claims 1 to 9, **characterized in that** said extract is obtained by supercritical carbon dioxide extraction.

**13.** Use according to one of claims 1 to 12, wherein said composition comprises from 0.001% to 10% by weight, and particularly from 0.02% to 1% by weight, of dry extract of said plant.

**14.** Use according to one of claims 1 to 13, **characterized in that** said composition is presented in any form appropriate for topical application, specifically in the form of a cream or gel, and particularly a cream or gel for the face, hands, bust or body.

**15.** Use according to one of claims 1 to 14, **characterized in that** said composition is formulated for topical application to the skin.